# EUROPÄISCHE PATENTSCHRIFT

(11) **EP 3 395 382 B1**
(45) Veröffentlichungstag und Bekanntmachung des Hinweises auf die Patenterteilung: **09.11.2022**
(21) Anmeldenummer: 18169603.0
(22) Anmeldetag: 26.04.2018
(51) Int. Cl.: A61M 1/16

(54) **VERFAHREN UND VORRICHTUNG ZUR INTERMITTIERENDEN, PULSIERENDEN PROPORTIONIERUNG EINER DIALYSIERFLÜSSIGKEITSMISCHUNG**
METHOD AND DEVICE FOR INTERMITTENT, PULSATING PROPORTIONING OF A DIALYSIS LIQUID MIXTURE
PROCÉDÉ ET DISPOSITIF DE PROPORTIONNEMENT PAR INTERMITTENCE DE MANIÈRE PULSATOIRE D'UN MÉLANGE DE FLUIDE DE DIALYSE

(30) Priorität: 27.04.2017 DE 102017109127
(43) Veröffentlichungstag der Anmeldung: 31.10.2018
(73) Patentinhaber: B. Braun Avitum AG, 34212 Melsungen (DE)
(72) Erfinder: Janik, Waldemar, 34212 Melsungen (DE); Ritter, Kai-Uwe, 34212 Melsungen (DE)
(74) Vertreter: Winter, Brandl - Partnerschaft mbB

(56) Entgegenhaltungen:
- EP-A1- 2 962 711
- WO-A1-2016/169642
- US-A1- 2014 220 699

## Beschreibung

Die Erfindung betrifft ein Verfahren zum Herstellen von Dialysierflüssigkeit, bei dem die basische und die saure Komponente dem Wasser abwechselnd oder zumindest einmalig zeitversetzt und intermittierend, pulsierend hinzugefügt werden, sodass aufgrund dieser Art der Zugabe eine einzelne Messeinrichtung genügt, um die Zusammensetzung der anzumischenden Dialysierflüssigkeit zu kontrollieren, wobei die Zugabe entweder durch zwei Pumpen, durch eine Pumpe samt Ventilen oder durch Unterdruck erfolgt.

### Hintergrund der Erfindung

Bei einer extrakorporalen Blutbehandlung, z. B. Hämodialyse, Hämofiltration, Hämodiafiltration etc., kommt eine Vorrichtung zur Herstellung von Dialysierflüssigkeit zum Einsatz, die die benötigte Dialysierflüssigkeit aus den Grundbestandteilen Wasser, basisches Fluid und saures Fluid herstellt. Die hergestellte Dialysierflüssigkeit wird während der Blutbehandlung eines Patienten durch die dialyseseitige Kammer eines Dialysators geleitet, über dessen semipermeable Membran durch Diffusion (Hämodialyse) oder Diffusion in Kombination mit Konvektion (Hämofiltration und Hämodiafiltration) Giftstoffe und Wasser aus dem Blut, das durch die blutseitige Kammer des Dialysators geleitet wird, aufgenommen werden.

Während der extrakorporalen Blutbehandlung wird das zu behandelnde Blut des Patienten in dem Dialysator von der Dialysierflüssigkeit umspült. Bei der basischen Komponente der Dialysierflüssigkeit handelt es sich gewöhnlicherweise um ein Substrat mit Natriumhydrogencarbonat (NaHCO₃) und bei der zweiten Komponente (SK) handelt es sich gewöhnlicherweise um eine Lösung mit Natriumchlorid (NaCl), Kaliumchlorid (KCl), Magnesiumchlorid (MgCl₂), Calciumchlorid (CaCl₂)), Glucose (C₆H₁₂O₆) und Essigsäure (CH₃COOH) und/oder Zitronensäure (C₆H₈O₇).

Zur Herstellung bzw. zur Proportionierung der Dialysierflüssigkeit werden in der Regel Dosierpumpen und Leitfähigkeitssonden eingesetzt. Dabei misst eine Sonde die Leitfähigkeit des NaHCO₃ nach Zugabe mittels einer ersten Dosierpumpe (BICLF). Nach Zugabe der sauren Komponente mittels einer weiteren Dosierpumpe misst eine weitere Sonde die Leitfähigkeit der gesamten Dialysierflüssigkeit (ENDLF).

Bei einer leitfähigkeitsgesteuerten Proportionierung werden die Zugabemengen anhand der gemessenen Leitfähigkeiten geregelt. Bei einer volumetrischen Proportionierung dienen die Leitfähigkeitssonden lediglich zur Kontrolle, da die Proportionierung direkt über die Dosierpumpenförderraten erfolgt. Dies erfordert jedoch die genaue Kenntnis der Zusammensetzung der eingesetzten Komponenten.

### Stand der Technik

Üblicherweise werden zur Herstellung von Dialysierflüssigkeit zwei Dosierpumpen und mindestens zwei Leitfähigkeitssonden eingesetzt. Dabei wird hochreinem Wasser über eine erste Dosierpumpe eine erste Komponente, basisch oder sauer, und über eine zweite Dosierpumpe die zweite Komponente, sauer oder basisch, zugemischt. Die Zugabe der jeweiligen Komponenten wird mit jeweils einer Leitfähigkeitssonde kontrolliert. Zur sicherheitstechnischen Überwachung der Zusammensetzung sind üblicherweise zusätzlich ein zweiter, unabhängiger Kanal und eine dritte Leitfähigkeitssonde vorhanden. Als Dosierpumpen kommen häufig Drehschieberkolbenpumpen und Membranpumpen zum Einsatz, die sich dadurch auszeichnen, dass sie nichtkontinuierlich fördern.

Der Einsatz mehrerer Dosierpumpen, gewöhnlich zwei, und mehrerer Leitfähigkeitssonden, gewöhnlich zwei bis drei, zum Anmischen von Dialysierflüssigkeit nach dem Verfahren der leitfähigkeitsgesteuerten Proportionierung ist mit hohen finanziellen Aufwendungen verbunden, da Dosierpumpen und Leitfähigkeitssonden hochpreisige Komponenten einer Vorrichtung zur extrakorporalen Blutbehandlung darstellen und ihre Lebensdauer darüber hinaus durch Verschleiß limitiert ist.

EP 2 962 711 A1 offenbart eine Dialysemaschine mit einer Aufbereitungsanlage für Dialysierflüssigkeit, die aus Wasser, einer sauren Komponente und einer basischen Komponente angemischt wird. Die saure Komponente und die basische Komponente münden an unterschiedlichen Mündungspunkten in die Hauptleitung. Die Leitfähigkeit des Gemischs wird jeweils hinter den einzelnen Mündungspunkten überprüft.

In der Patentanmeldung WO 2016/169642 A1 wird zudem eine Vorrichtung und ein Verfahren zur Herstellung einer medizinischen Mischlösung aus zwei flüssigen Komponenten offenbart, wobei die Fördermittel zur Förderung der beiden Komponenten derart betrieben werden, dass eine Modulation der Konzentration der ersten und zweiten Komponente erfolgt, und an einer Messstelle die Leitfähigkeit oder ein anderer mit der Leitfähigkeit korrelierter Parameter der Mischlösung gemessen wird und die Modulation der Konzentrationen in einem Sollzustand derart erfolgt, dass keine Modulation oder eine bestimmte Modulation der gemessenen Leitfähigkeit oder des mit der Leitfähigkeit korrelierten Parameters auftritt.

### Kurze Beschreibung der Erfindung

Aufgabe der vorliegenden Erfindung ist es daher, die bekannten Nachteile bei der Herstellung von Dialysierflüssigkeit für die extrakorporale Blutbehandlung zu überwinden und für die Herstellung von Dialysierflüssigkeit aus hochreinem Wasser, einer basischen und einer sauren Komponente ein Verfahren und eine Vorrichtung bereitzustellen, die sich dadurch auszeichnen, dass die Anmischung und Proportionierung der Dialysierflüssigkeit deutlich effizienter durchgeführt wird und somit die einmaligen und laufenden Kosten für die Vorrichtung und das Verfahren erheblich gesenkt werden.

Diese Aufgabe wird erfindungsgemäß durch die Merkmalskombination des Anspruchs 1 in Verbindung mit der Merkmalskombination des Anspruchs 13 gelöst. Vorteilhafte Weiterbildungen der Erfindung sind Gegenstände der beigefügten Unteransprüche.

Ein Grundgedanke der Erfindung besteht somit darin, den erforderlichen technischen Aufwand bei der Konstruktion einer Vorrichtung und Durchführung eines Verfahrens zur Herstellung von Dialysierflüssigkeit, die bei extrakorporalen Blutbehandlungen zum Einsatz kommt, bei gleichbleibender technischer Zuverlässigkeit zu reduzieren.

In anderen Worten ausgedrückt kommt die erfindungsgemäße Vorrichtung im Vergleich zum Stand der Technik mit einer geringeren Anzahl an teuren sensorischen Komponenten aus und das erfindungsgemäße Verfahren ist so konzipiert, dass das Mess- und Steuerungsvermögen der verbauten Komponenten mit Unterstützung durch mathematische Methoden so effizient ausgenutzt wird, dass eine Mehreinsatz an technischen Mess- und Steuerungsmitteln überflüssig ist bzw. eine weitere Messeinrichtung vollständig ersetzt wird.

Man kann auch sagen, dass die Gesamtzahl an erforderlichen Messeinrichtungen, beispielsweise Mess-Sensoren, reduziert wird bzw. eine im Stand der Technik übliche Mehrzahl an Messeinrichtung ersetzt wird durch geschickte Anordnung einer einzigen Messeinrichtung kombiniert mit einer entsprechenden Verfahrensführung.

Konkret ist die Verschlankung der technischen Ausstattung einer Vorrichtung zur Herstellung von Dialysierflüssigkeit derart vorgesehen, dass zur Kontrolle und Steuerung des Verfahrens zur Herstellung von Dialysierflüssigkeit nur eine einzige Messeinrichtung zur Aufnahme mindestens eines physikalischen und/oder chemischen Parameters, z. B. eine Leitfähigkeitssonde, eingesetzt wird. Bei Bedarf kann die technische Ausrüstung der Vorrichtung weiter reduziert werden, indem zur Förderung der erforderlichen Dialysierflüssigkeitskomponenten, nämlich hochreines oder osmotisches Wasser (nachfolgend als Wasser bezeichnet) und eine basische Komponente (nachfolgend auch basisches Fluid genannt) und eine saure Komponente (nachfolgend auch saures Fluid genannt), maximal eine (Dosier)Pumpe eingesetzt wird. Die Zugabe der beiden Fluide in das Wasser, also die Herstellung der Dialysierflüssigkeit, erfolgt durch den Betrieb einer (ggf. einzigen) (Dosier)Pumpe und/oder der Steuerung von Ventilen und wird abwechselnd, zumindest aber einmalig zeitversetzt durchgeführt. Aufgrund dieses Verfahrens ist es möglich, die Messung des mindestens einen physikalischen und/oder chemischen Parameters des Wasser-Fluid-Gemischs (auch als Komponentengemisch bezeichnet) mit einer einzigen Messvorrichtung durchzuführen, da in der Messeinrichtung für das Wasser-Fluid-Gemisch nach Zugabe zunächst eines ersten Fluids der basischen und sauren Fluide in das Wasser unter Abgleich des gemessenen Ist-Werts mit vorgegebenen Sollwerten die Dosierung bzw. Förderrate dieses ersten Fluids kontrolliert und justiert bzw. gesteuert werden kann und für das Wasser-Fluid-Gemisch, das aus Wasser und dem zugebenen ersten Fluid, z. B. ein Referenzwert ermittelt werden kann. Nach bevorzugter Aufnahme des Referenzwerts kann das zweite Fluid der basischen und sauren Fluide in das Wasser-Fluid-Gemisch gegeben werden, entweder im Wechsel mit dem ersten Fluid, wobei in diesem Fall kein Referenzwert erforderlich ist, da beide Fluid-WasserGemische einzeln und separat eingemessen werden, oder zusätzlich zum ersten Fluid unter Abgleich mit dem Referenzwert, ohne dass die Kontrolle über das Wasser-Fluid-Gemisch mit einer einzigen Messeinrichtung eingeschränkt wird.

Insgesamt werden erfindungsgemäße Vorteile dahingehend erzielt, dass gegenüber den bekannten Vorrichtungen und Verfahren zur Herstellung von Dialysierflüssigkeit für den Einsatz bei einer extrakorporalen Blutbehandlung auf mindestens eine Messeinrichtung verzichtet werden kann, was den Aufwand bei der Bereitstellung einer Vorrichtung zur Herstellung von Dialysierflüssigkeit ökonomischer macht und somit auch die Kosten des Verfahrens senkt. Da Sensoren teuer in der Anschaffung sind, fällt die finanzielle Ersparnis bei Verzicht auf mindestens eines dieser Teile im Verhältnis zu den Gesamtkosten beträchtlich aus. Darüber hinaus müssen Sensoren gewartet und von Zeit zu Zeit ersetzt werden, sodass neben den monetären Sachaufwänden auch der personelle zeitliche Betreuungsaufwand reduziert wird. Weiter bedeutet der Wegfall von Komponenten eine Verminderung von Toträumen und eine Verringerung des Risikos auf verschleißbedingten Ausfall der Vorrichtung.

Die Aufgabe wird gelöst durch eine Vorrichtung zur Herstellung von Dialysierflüssigkeit für den Einsatz bei einer extrakorporalen Blutbehandlung mit einer Hauptleitung zur Zufuhr von Wasser, vorzugweise osmotisches oder hochreines Wasser, in deren Verlauf ein saures Fluid und ein basisches Fluid in jeweils einer bestimmten Dosierung bzw. Förderrate eingeleitet wird, wobei die Dosierung oder Förderrate von einer Steuer- und Regeleinheit in Abhängigkeit von mindestens einem chemischen und/oder physikalischen Parameter, vorzugsweise die Leitfähigkeit, des Wasser-Fluid-Gemischs eingestellt wird und der mindestens eine chemische und/oder physikalische Parameter von einer Messeinrichtung, insbesondere einer Leitfähigkeitsmesssonde, erfasst wird, dadurch gekennzeichnet, dass eine erste, vorzugweise einzige, Messeinrichtung an einem Abschnitt der Hauptleitung, der jeweils stromab zu der Einleitstelle für das saure Fluid und zu der Einleitstelle für das basische Fluid liegt, angeordnet ist und die Steuer- und Regeleinheit dafür ausgelegt ist, die Einleitung des sauren Fluids und des basischen Fluids zumindest temporär (für den Einmessvorgang) so zu steuern, dass über einen vorbestimmten Zeitraum oder ein vorbestimmtes Intervall nur ein Fluid aus den sauren und basischen Fluiden in die Hauptleitung eingeleitet wird.

In anderen Worten wird die Aufgabe gelöst durch eine Vorrichtung zur Herstellung von Dialysierflüssigkeit, welche ein Wasser-Fluid-Gemisch bestehend aus Wasser und einem basischen Fluid und einem sauren Fluid darstellt, für den Einsatz bei einer extrakorporalen Blutbehandlung mit einer (einzigen) Messeinrichtung zur Erfassung mindestens eines physikalischen und/oder chemischen Parameters des Wasser-Fluid-Gemischs, wobei die Messeinrichtung zumindest am Anfang des Verfahrens (Einmessvorgangs) mindestens einen Parameter eines Wasser-Fluid-Gemischs zunächst bei einer isolierten Zugabe eines ersten Fluids aus den sauren und basischen Fluiden in das Wasser ermittelt und basierend darauf die Dosierung/Förderrate dieses ersten Fluids optimal einstellt. Anschließend kann für das Wasser-Fluid-Gemisch, das zu diesem Zeitpunkt aus zwei Komponenten besteht, vorzugsweise ein Referenzwert ermittelt werden, bevor das zweite Fluid aus den sauren und basischen Fluiden in das Wasser-Fluid-Gemisch eingeleitet und mindestens ein Parameter, der mit dem Referenzwert vergleichbar ist um Abweichungen infolge der Zugabe des Fluids zu bestimmen und dessen Dosierung/Förderrate entsprechend einzustellen, des Wasser-Fluid-Gemischs, das dann aus drei Komponenten besteht, ermittelt wird. Alternativ ist es aber auch möglich, die Förderung des ersten Fluids zu unterbrechen und das zweite Fluid zu fördern, um dessen Dosierung/Förderrate separat optimal einzustellen, worauf dann das erste Fluid mit der zuvor eingestellten Förderrate wieder zugeschaltet wird. Damit ist es möglich mit einer einzigen Messeinrichtung die Mischung aus Wasser mit zwei Fluiden zu kontrollieren. Die Förderung des Wasser-Fluid-Gemischs bzw. dessen einzelner Komponenten kann über bekannte Fördermittel wie Pumpen, ggf. in Kombination mit Ventilen, erfolgen.

Es ist bereits ausreichend und bevorzugt vorgesehen, dass an der Vorrichtung eine einzige Pumpe zur Förderung des basischen Fluids und des sauren Fluids angeordnet ist. Dies kann beispielsweise durch einen gemeinsamen Leitungsabschnitt erfolgen. Auch die Förderung des Wassers und beider Fluide kann über eine einzige Pumpe, ggf. in Kombination mit einem angelegten Unterdruck auf der Hauptleitung oder durch Reduktion des Leitungsquerschnitts der Hauptleitung, erfolgen. Dazu kann für das Einleiten des sauren Fluids und/oder das Einleiten des basischen Fluids in die Hauptleitung bevorzugt mindestens ein Ventil, vorzugsweise ein Mehrwegeventil vorgesehen sein. Auch kann die Hauptleitung mit einem Ventil, vorzugsweise ein Druckregelventil, versehen sein, das insbesondere stromauf von der am weitesten stromauf gelegenen Einleitstelle für das saure Fluid und das basische Fluid angeordnet ist.

Vorteilhafterweise kann somit mindestens eine Messeinrichtung und mindestens eine Pumpe eingespart werden. Die Ventile ermöglichen bei reduzierter Pumpenzahl individuelle Förderraten für die Komponenten des Wasser-Fluid-Gemischs, da die Förderrate der gemeinsamen Pumpe und die Ventilöffnungs- und schließzeiten der Ventile entsprechend kombiniert werden können.

Weiter bevorzugt kann eine weitere Messeinrichtung unmittelbar stromab zu der ersten Messeinrichtung vorgesehen und dazu angepasst sein, mindestens einen physikalischen und/oder chemischen Parameter des die Hauptleitung durchströmenden Wasser-Fluid-Gemischs, zu erfassen.

Um auffällige bzw. fehlerhafte Messergebnisse schnell und zuverlässig als solche zu identifizieren, werden die an der ersten Messeinrichtung gemessenen Parameter mit den an der weiteren Messeinrichtung gemessenen Parametern verglichen. Dabei ist die weitere Messeinrichtung mit einer separaten Überwachungseinheit verbunden. In anderen Worten ausgedrückt werden die Messergebnisse der ersten Messeinrichtung durch eine zweite Messung an der weiteren Messeinrichtung kontrolliert und somit die Sicherheit bei der Herstellung der Dialysierflüssigkeit erhöht.

Außerdem bevorzugt kann die Vorrichtung mindestens eine Mischeinrichtung, vorzugsweise einen statischer Mischer oder eine Kammer eines kammerbasierten Bilanzierungssystems, aufweisen und die Mischeinrichtung vorzugsweise stromab zu der ersten und/oder weiteren Messeinrichtung und weiter vorzugsweise jeweils stromab zu der Einleitstelle für das saure Fluid und zu der Einleitstelle für das basische Fluid angeordnet sein.

Die Durchmischung des Wasser-Fluid-Gemischs nach Zugabe des basischen Fluids und/oder sauren Fluids in einer Mischeinrichtung führt zu einem repräsentativeren, also genaueren, Messergebnis an der Messeinrichtung, da Konzentrationsschwankungen infolge von Diffusion nach der Zugabe des basischen Fluids und/oder sauren Fluids in das Wasser bei der Mischung homogenisiert, also ausgeglichen werden. Bei dem eingesetzten Mischer kann es sich um einen statischen Mischer (Statikmischer), wie beispielsweise einen Kenics-Mischer, oder um die Kammer eines kammerbasierten Bilanzierungssystems handeln. Eine Vermischung bzw. Homogenisierung des Fluid-Gemischs unmittelbar stromabwärts der Zugabestelle(n) begünstigt die Genauigkeit der Messergebnisse der Messeinrichtung, da sich Turbulenzen, die beim Mischvorgang entstanden sind, über eine möglichst lange Strömungsstrecke zwischen dem Austritt aus der Mischeinrichtung und dem Passieren der Messeinrichtung bestmöglich auflösen. Die Anordnung stromab der weiteren Messeinrichtung ist insbesondere von Vorteil, wenn die Zugabe des basischen Fluids und/oder des sauren Fluids kodiert sind, beispielsweise über die Leitfähigkeit. Das basische Fluid weist typischerweise eine niedrigere Leitfähigkeit als das saure Fluid auf. Damit die Leitfähigkeit des die Messeinrichtung passierenden Fluid-Gemischs, insbesondere der zeitliche Verlauf der Leitfähigkeit, möglichst genau erfasst werden kann, sollte das Wasser-Fluid-Gemisch vor Passieren der Messeinrichtung keine Mischeinrichtung durchlaufen. Um jedoch Konzentrationsschwankungen für den weiteren Verfahrensverlauf bzw. Gebrauch der hergestellten Dialysierflüssigkeit möglichst zu reduzieren, ist in seinem solchen Fall eine Vermischung nach Austritt des Fluids-Gemischs aus der Messeinrichtung vorgesehen.

Weiter betrifft die Erfindung ein Verfahren zur Herstellung von Dialysierflüssigkeit, die die drei Komponenten Wasser und basisches Fluid und saures Fluid enthält, für den Einsatz bei einer extrakorporalen Blutbehandlung an einer Vorrichtung zur extrakorporalen Blutbehandlung mit den Schritten
- Fördern von Wasser, vorzugsweise osmotisches oder hochreines Wasser, über eine Hauptleitung,
- Fördern eines basischen Fluids und Einleiten an einer Einleitstelle in die Hauptleitung,
- Fördern eines sauren Fluids und Einleiten an einer Einleitstelle in die Hauptleitung
- Messen mindestens eines physikalischen und/oder chemischen Parameters, vorzugsweise Leitfähigkeit und besonders bevorzugt temperaturkompensierte Leitfähigkeit, des Wasser-Fluid-Gemischs, das aus Wasser, dem sauren Fluid und/oder dem basischen Fluid besteht, mittels einer ersten Messeinrichtung, vorzugsweise einer Leitfähigkeitssonde und besonders bevorzugt einer temperaturkompensierten Leitfähigkeitssonde, und
- Einstellen der Förderraten für das Wasser, das saure Fluid und/oder das basische Fluid durch eine Steuer- und Regeleinheit in Abhängigkeit vom erfassten Messwert, dadurch gekennzeichnet dass das Messen des mindestens einen physikalischen und/oder chemischen Parameters mittels einer vorzugsweise einzigen Messeinrichtung erfolgt, die an einem Abschnitt der Hauptleitung, der jeweils stromab zu der Einleitstelle für das saure Fluid und zu der Einleitstelle für das basische Fluid liegt, angeordnet wird und die Steuer- und Regeleinheit das Einleiten des sauren Fluids und/oder basischen Fluids zumindest temporär so steuert, dass über einen vorbestimmten Zeitraum oder ein vorbestimmtes Intervall nur eines aus den sauren und basischen Fluiden in die Hauptleitung eingeleitet und der Parameter für ein Einstellen der Dosierung/Förderrate dieses einen Fluids getaktet oder kontinuierlich gemessen wird.

Bevorzugt wird der Parameter des Wasser-Fluid-Gemischs mit nur dem einen Fluid (dem ersten Fluid) gemessen und basierend darauf die Dosierung oder Förderrate dieses einen Fluids (des ersten Fluids) bestimmt, worauf die Einleitung dieses einen Fluids (des ersten Fluids) unterbrochen und stattdessen das andere Fluid (das zweite Fluid) aus den basischen und sauren Fluiden eingeleitet wird, wobei der Parameter des Wasser-Fluid-Gemischs mit nur dem anderen Fluid (dem zweiten Fluid) gemessen und basierend darauf die Dosierung oder Förderrate dieses anderen Fluids (des zweiten Fluids) bestimmt wird und schließlich beide Fluide (das erste Fluid und das zweite Fluid) mit den zuvor bestimmten Dosierungen oder Förderraten eingeleitet werden. Alternativ bevorzugt wird der Parameter des Wasser-Fluid-Gemischs mit nur dem einen Fluid (dem ersten Fluid) gemessen und basierend darauf die Dosierung oder Förderrate dieses einen Fluids (des ersten Fluids) bestimmt und eingestellt wird, wobei der zugehörige Parameter als Referenzwert festgehalten wird, worauf das andere Fluid (das zweite Fluid) dem Wasser-Fluid-Gemisch zugeschaltet wird und die Parameter-Abweichung vom Referenzwert ermittelt sowie basierend darauf die Dosierung oder Förderrate des anderen Fluids (des zweiten Fluids) bestimmt und eingestellt wird.

In anderen Worten ausgedrückt wird bei dem erfindungsgemäßen Verfahren zur Herstellung von Dialysierflüssigkeit für den Einsatz bei einer extrakorporalen Blutbehandlung an einer Vorrichtung zur extrakorporalen Blutbehandlung die Zusammensetzung des Wasser-Fluid-Gemischs, das bestehend aus hochreinem Wasser, einer basischen und einer sauren Komponente der Dialysierflüssigkeit entspricht, anhand des mindestens einen gemessenen physikalischen und/oder chemischen Parameters kontrolliert und gesteuert, wobei erst ein erstes Fluid aus den basischen und sauren Fluiden isoliert in das Wasser eingeleitet und das Wasser-Fluid-Gemisch aus zwei Komponenten, nämlich Wasser und dem ersten Fluid aus den basischen und sauren Fluiden, gemessen wird, bevor das zweite Fluid aus den basischen und sauren Fluid in das Wasser bzw. das Wasser-Fluid-Gemisch aus zwei Komponenten eingeleitet und gemessen wird. Die Messung des mindestens einen physikalischen und/oder chemischen Parameters des Wasser-Fluid-Gemischs, das aus Wasser, dem basischen Fluid und/oder dem sauren Fluid besteht, erfolgt dabei ausschließlich an einer Strömungsstelle, die in Strömungsrichtung des Wassers stromab der Einleitstelle für das basische Fluid und der Einleitstelle für das saure Fluid in die Hauptleitung liegt.

Die Zugabe der Fluide in das Wasser bzw. Wasser-Fluid-Gemisch erfolgt dabei bevorzugt intermittierend pulsierend, die Fluide werden also stoßweise mit Unterbrechungen gefördert. Dies wirkt sich vorteilhaft auf die Vermischung mit dem Wasser bzw. Wasser-Fluid-Gemisch aus. Weiter genügt aufgrund dieser Art der Zugabe eine einzelne Messeinrichtung, um die Zusammensetzung der anzumischenden Dialysierflüssigkeit zu kontrollieren. Vorteilhafterweise kann dadurch mindestens eine, im Stand der Technik übliche, Messeinrichtung eingespart werden.

Bevorzugt können das Fördern und Einleiten des basischen Fluids (mit der zuvor bestimmten Dosierung oder Förderrate) und das Fördern und Einleiten des sauren Fluids (mit der zuvor bestimmten Dosierung oder Förderrate) abwechselnd, insbesondere kontinuierlich abwechselnd, erfolgen. Bevorzugt können die Förderraten des Wassers und/oder des geförderten Fluids mit jedem Zyklus und besonders bevorzugt zu jedem Zeitpunkt der Förderung, neu eingestellt bzw. angepasst werden. Auch kann während der Änderung der Förderrate einer Gemischkomponente (z.B. das geförderte Fluid) die zuvor eingestellte Förderrate der mitgeförderten Komponente (z.B. Wasser) beibehalten werden.

Alternativ bevorzugt können das Fördern des basischen Fluids (mit der zuvor bestimmten Dosierung oder Förderrate) und das Fördern des sauren Fluids (mit der zuvor bestimmten Dosierung oder Förderrate) zeitversetzt parallel erfolgen.

Durch den Förderbeginn und das zunächst isolierte Einleiten erst eines Fluids aus den basischen und sauren Fluiden in das Wasser und Anpassen der Förderrate dieses einen Fluids und/oder des Wassers auf einen definierten Sollwert für das Wasser-Fluid-Gemisch kann die erforderliche Förderrate des einen Fluids und/oder des Wassers zuverlässiger und schneller berechnet und/oder eingestellt werden, als bei gleichzeitiger Förderung bzw. gleichzeitigem Förderbeginn von basischen und sauren Fluid und Einleiten in das Wasser. Nachdem das erste Fluid aus den basischen und sauren Fluiden in das Wasser eingeleitet und das Wasser-Fluid-Gemisch gemessen wurde, kann als eine erste Alternative ein Referenzwert für diese eine Fluidzugabe festgehalten werden. Anschließend kann das zweite Fluid dem zuvor eingemessenen Wasser-Fluid-Gemisch zusätzlich zugegeben und die dadurch resultierenden Parameterabweichungen vom Referenzwert erfasst werden, die als Basis für die Dosierung/Förderrate des zweiten Fluids verwendet werden.

Als zweite Alternative kann die Förderung des ersten Fluids nach erfolgter Einstellung von dessen Dosierung/Förderrate unterbrochen und durch die Förderung des zweiten Fluids ersetzt werden. Sobald dessen Dosierung/Förderrate durch Messen des Parameters eingestellt ist, kann das erste Fluid mit zuvor eingestellter Dosierung/Förderrate wieder zugeschaltet werden. Dadurch können bei anschließendem Einleiten des zweiten Fluids aus den basischen und sauren Fluiden in das Wasser-Fluid-Gemisch die Förderraten aller drei Gemischkomponenten, nämlich Wasser, das basische Fluid und das saure Fluid, mit Hilfe einer einzigen Messeinrichtung kontrolliert werden.

Bevorzugt kann bei einer Anpassung einer Förderrate eines Fluids aus den basischen und sauren Fluiden für den Zeitraum des Anpassens, insbesondere von der Änderung der Förderrate bis zum Erreichen des neuen Sollwerts des Wasser-Fluid-Gemischs, die vor der Anpassung eingestellte Förderrate des anderen Fluids aus den basischen und sauren Fluiden beibehalten oder dessen Förderung pausiert werden.

Besonders bevorzugt kann die Anpassung einer Förderrate einer Komponente unter Beibehaltung der Förderraten der gleichzeitig geförderten anderen Komponenten erfolgen. Dies betrifft insbesondere den Fall der parallelen Förderung der beiden Fluide.

Die zu ändernde Förderrate kann zuverlässiger und schneller eingestellt werden, wenn während der Neueinstellung die neu einzustellende Förderrate die einzige geänderte Größe im Wasser-Fluid-Gemisch darstellt. Dadurch ist es auch möglich, eine Förderrate für eine Komponente anzupassen während die Förderung der beiden anderen Komponenten mit den zuvor eingestellten Förderraten weiter betrieben wird. Dies betrifft insbesondere den Fall der parallelen Förderung der beiden Fluide. Erfindungsgemäß schließt der Vergleich mit dem Sollwert eine Regelung mit ein. Bei der Regelung werden Ist- und Sollwert miteinander verglichen. Die Regelabweichung wird gebildet und anschließend an einen Regler übergeben, der dann eine entsprechende Stellgröße (hier eine Pumpenförderrate) ermittelt. Als Regler kommen bspw. unstetige Mehrpunkt-Regler, P- Regler, PI- Regler, PID- Regler, Fuzzy- Regler, adaptive Regler, hybride Regler und/oder Regler basierend auf künstlichen neuronalen Netzen in Frage. Die Ermittlung der Soll-Förderraten kann durch Berechnung oder auch durch analytische Verfahren erfolgen.

Weiter bevorzugt kann das Wasser-Fluid-Gemisch bei einer Änderungen oder neuen Einstellung mindestens eines Sollwerts für das Wasser-Fluid-Gemisch bis zum wenigstens erstmaligen Erreichen des Sollwerts an der Vorrichtung zur extrakorporalen Blutbehandlung vorbeigeführt werden. Dies kann auch zur isolierten Kontrolle der einzelnen Dosierungen bzw. Förderraten in regelmäßigen Abständen angewandt werden. Dadurch kann eine sonst drohende Alkalose oder Azidose des Patienten verhindert werden. Nach einer solchen kurzzeitigen Unterbrechung der Förderung und Zugabe des basischen und/oder sauren Fluids kann dasjenige, dessen Förderung und Zugabe unterbrochen war, in dem Maß zusätzlich gefördert und zugegeben werden, dass sich im langfristigen Mittel für das Fluid-Gemisch weiterhin die zu erzielende physiologische Zusammensetzung ergibt.

Als Sollwerte können grundsätzliche alle relevanten Verfahrensparameter in Betracht kommen, beispielsweise Mischverhältnisse der Komponenten, Elektrolytkonzentrationen im Gemisch, ph-Wert des Gemischs, Pumpvolumen und/oder Fördervolumen.

Bevorzugt kann in einem Fall, in dem zum Erreichen mindestens eines Sollwerts des Wasser-Fluid-Gemischs eine definierte Zeitspanne überschritten wird und/oder eine Förderrate eingestellt wird, die einen definierten Grenzwert überschreitet, ein Warnhinweis an der Vorrichtung zur extrakorporalen Blutbehandlung ausgegeben wird.

Auf diese Weise kann sichergestellt werden, dass nicht versehentlich ein falsches Fluid an die Vorrichtung angeschlossen wurde und dass der Anschluss des Fluids korrekt erfolgt ist. Weiter kann auf dieser Weise erkannt werden, ob der Vorlagebehälter, beispielsweise ein Kanister, des angeschlossenen Fluids leer ist und ersetzt werden muss. Ein Warnsignal kann beispielsweise akustisch, visuell oder haptisch erfolgen.

Weiter bevorzugt kann das Verfahren weiter einen Schritt Messen mindestens eines physikalischen und/oder chemischen Parameters des Wasser-Fluid-Gemischs, das aus Wasser, dem basischen Fluid und/oder dem sauren Fluid besteht, mittels einer weiteren Messeinrichtung, vorzugsweise eine Leitfähigkeitssonde und besonders bevorzugt eine temperaturkompensierte Leitfähigkeitssonde, die an der Hauptleitung unmittelbar stromab zu der ersten Messeinrichtung angeordnet ist, aufweist und dass in einem Fall, in dem eine Abweichung zwischen dem an der ersten Messeinrichtung und dem an der weiteren Messeinrichtung gemessenen Parameter einen definierten Grenzwert überschreitet, ein Warnhinweis an der Vorrichtung zur extrakorporalen Blutbehandlung ausgegeben wird.

Eine weitere Messung, man kann auch sagen eine Kontrollmessung, dient zur Überprüfung der Messung, die an der ersten Messeinrichtung vorgenommen wurde, und soll die Sicherheit bei der Herstellung des Wasser-Fluid-Gemischs bzw. der Dialysierflüssigkeit erhöhen. Denkbare erste und weitere Messeinrichtungen sind neben Leitfähigkeitssonden, bevorzugt mit solche mit Temperatursensor, auch ionenselektive Elektroden oder optische Messmittel, beispielsweise Einrichtungen zur laserinduzierten Plasmaspektroskopie.

Außerdem bevorzugt kann das Wasser-Fluid-Gemisch, das aus Wasser, dem basischen Fluid und/oder dem sauren Fluid besteht, mittels mindestens einer Mischeinrichtung, vorzugsweise einem statischer Mischer, durchmischt werden.

Dies dient der Homogenisierung des Fluid-Gemischs und führt zu genaueren und zuverlässigeren Messergebnissen. Denkbare Mischeinrichtungen sind beispielsweise Kenics-Mischer oder Kammern von kammerbasierten Bilanzierungssystemen. Wenn das Verfahren ohne Mischer durchgeführt wird, kann zur genaueren Bestimmung des Messwerts eine Mittelwertbildung bzw. Filterung der gemessenen Parameter, z. B. Leitfähigkeit, mittels analoger oder digitaler Filterung erfolgen.

Bevorzugt kann die Mischeinrichtung eine Kammer eines kammerbasierten Bilanzierungssystems sein und der physikalische und/oder chemische Sollwert eines Wasser-Fluid-Gemischs, das aus Wasser, dem basischen Fluid und/oder dem sauren Fluid besteht, so definiert sein, dass er rechnerisch innerhalb einer Zeiteinheit, die höchstens einer Bilanzkammerumschaltung entspricht, realisiert werden kann. Dadurch kann eine Durchmischung in der Bilanzkammer besser realisiert werden. In Fällen, in denen der physikalische und/oder chemische Sollwert nicht innerhalb einer Zeiteinheit, die höchstens einer Bilanzkammerumschaltung entspricht, realisiert werden kann, kann der Sollwert in mehreren Stufen angefahren werden, wobei eine einzelne Sollwert-Stufe so definiert sein kann, dass sie rechnerisch innerhalb einer Zeiteinheit, die höchstens einer Bilanzkammerumschaltung entspricht, realisiert werden kann. Besonders bevorzugt kann die Zugabe des basischen Fluids und des sauren Fluids kodiert werden, vorzugsweise durch Verwendung eines Barker-Codes.

Bei der Zugabe des basischen Fluids ist die zu erzielende Leitfähigkeit bzw. die zu erzielende Natriumkonzentration an der Messeinrichtung niedriger als die zu erzielende Leitfähigkeit bzw. zu erzielende Natriumkonzentration bei Zugabe des sauren Fluids. Das Signal bzw. der Messwert des basischen Fluids kann dann entsprechend dem Barker-Code mit "-1" und das Signal bzw. der Messwert des sauren Fluids mit "+1" kodiert werden. Das an der Messeinrichtung resultierende Signal kann dann mit entsprechenden mathematischen Mitteln verarbeitet bzw. entfaltet werden, woraufhin Rückschlüsse auf die Zugabemengen der einzelnen Fluide gezogen werden können.

Die soeben beschriebenen Verfahren beruhen auf der leitfähigkeitsgesteuerten Proportionierung des Fluid-Gemischs bzw. der Dialysierflüssigkeit. Es ist selbstverständlich auch möglich, die Dialysierflüssigkeit nach dem Prinzip der volumetrischen Proportionierung herzustellen, sofern die Pumpenförderraten und die Zusammensetzung der Fluide genau bekannt sind. Die Messung des mindestens einen physikalischen und/oder chemischen Parameters erfolgt auch bei diesem Prinzip mit nur einer Messeinrichtung. Der gemessene mindestens eine physikalische und/oder chemische Parameter dient hierbei lediglich als Kontrolle und nicht als Ist-Werte einer Regelung.

### Kurzbeschreibunq der Zeichnungen

Die Erfindung wird nachstehend anhand bevorzugter Ausführungsbeispiele unter Bezugnahme auf die beigefügten Zeichnungen näher beschrieben.
Fig. 1 zeigt eine schematische Darstellung einer ersten erfindungsgemäßen Vorrichtung.
Fig. 2 zeigt eine schematische Darstellung einer zweiten erfindungsgemäßen Vorrichtung.
Fig. 3 zeigt eine schematische Darstellung einer dritten erfindungsgemäßen Vorrichtung.
Fig. 4 zeigt ein Diagramm einer pulsierenden Pumpenförderung aus dem Stand der Technik.
Fig. 5 zeigt eine schematische Darstellung des Verfahrens gemäß einem Aspekt der Erfindung.
Fig. 6 zeigt eine schematische Darstellung eines ersten erfindungsgemäßen Modus der Zudosierung.
Fig. 7 zeigt eine schematische Darstellung eines zweiten erfindungsgemäßen Modus der Zudosierung.
Fig. 8 zeigt eine schematische Darstellung eines erfindungsgemäßen Modus der Förderratenanpassung.
Fig. 9 zeigt ein Diagramm eines Dosierungs-/Zugabeverfahrens und ein Diagramm des aufgenommen Messsignals gemäß einem Aspekt der Erfindung.
Fig. 10 zeigt ein Diagramm eines kodierten Dosierungs-/Zugabeverfahrens und ein Diagramm des aufgenommen Messsignals gemäß einem Aspekt der Erfindung.

### Detaillierte Beschreibung bevorzugter Ausführungsbeispiele

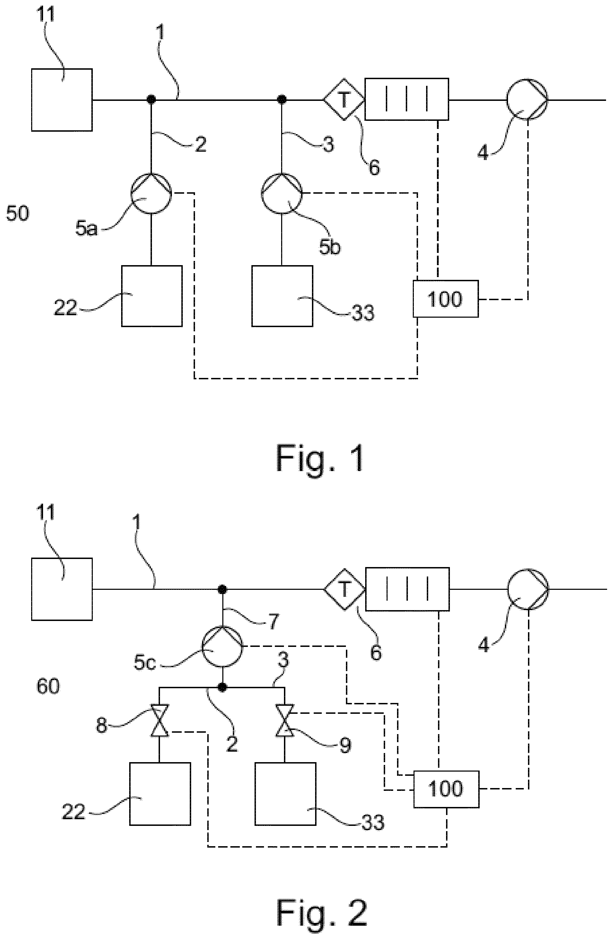

Fig. 1 zeigt eine erste erfindungsgemäße Ausführungsform der Vorrichtung zur Herstellung von Dialysierflüssigkeit. Eine (erste) ggf. einstellbare Pumpe 4 fördert/saugt Wasser aus einem Reservoir 11, das ein Behälter wie beispielsweise ein Kanister oder eine kontinuierliche Quelle sein kann, über eine Hauptleitung 1 in Richtung der Pumpe 4. Eine (zweite) (Dosier)pumpe 5a, die an einer Zuleitung 2 angeordnet ist, fördert ein basisches Fluid aus einem Vorlagebehälter 22, der u.a. ein Kanister oder eine Kartusche sein kann, über die Zuleitung 2 zur Hauptleitung 1 hin, wo die Zuleitung 2 in die Hauptleitung 1 mündet. Eine (dritte) (Dosier)pumpe 5b, die an einer weiteren Zuleitung 3 angeordnet ist, fördert ein saures Fluid aus einem Vorlagebehälter 33, der u. a. ein Kanister sein kann, über die Zuleitung 3 zur Hauptleitung 1 hin, wo die Zuleitung 3 in die Hauptleitung mündet. Die Zugabe des basischen Fluids und sauren Fluids in das Wasser erfolgt über Betreiben der (Dosier)pumpen 5a und 5b. Stromabwärts der Mündungsstelle der Zuleitung 3 in die Hauptleitung 1 ist eine Messeinrichtung 6, die u.a. eine Leitfähigkeitssonde, bevorzugt eine Leitfähigkeitssonde mit Temperatursensor zur temperaturkompensierten Leitfähigkeitsbestimmung, sein kann, angeordnet. Die Messeinrichtung 6 misst mindestens einen physikalischen und/oder chemischen Parameter des vorbeiströmenden Wasser-Fluid-Gemischs. Durch eine Steuer- und Regeleinheit 100 werden die Signale der Messeinrichtung 6 verarbeitet und die Pumpen 4, 5a und 5b gesteuert.

Fig. 2 zeigt eine zweite erfindungsgemäße Ausführungsform der Vorrichtung zur Herstellung von Dialysierflüssigkeit. Die Grundstruktur und die Referenzzeichen aus der ersten Ausführungsform bleiben unverändert, sodass im Folgenden nur auf Unterschiede der ersten und zweiten Ausführungsform eingegangen wird. Anstelle der (Dosier)pumpen 5a und 5b fördert eine (zweite) (Dosier)pumpe 5c das basische Fluid und das saure Fluid zur Hauptleitung 1 hin, wobei die Zuleitung 2 und die Zuleitung 3 zu einem gemeinsamen Leitungsabschnitt 7 zusammenlaufen, an dem die Pumpe 5c angeordnet ist, und der gemeinsame Leitungsabschnitt 7 in die Hauptleitung 1 mündet. An der Zuleitung 2 ist ein (steuerbares) Ventil 8 und an der Zuleitung 3 ist ein (steuerbares) Ventil 9 vorgesehen. Die Zugabe des basischen Fluids und sauren Fluids in das Wasser erfolgt über Betreiben der (Dosier)pumpe 5c in Kombination mit dem Öffnen und Schließen der Ventile 8 und 9. Stromab von der Mündungsstelle des gemeinsamen Leitungsabschnitts 7 in die Hauptleitung 1 ist die Messeinrichtung 6 angeordnet. Durch eine Steuer- und Regeleinheit 100 werden die Signale der Messeinrichtung 6 verarbeitet und die Pumpen 4 und 5c sowie ggf. die Ventile 8 und 9 gesteuert.

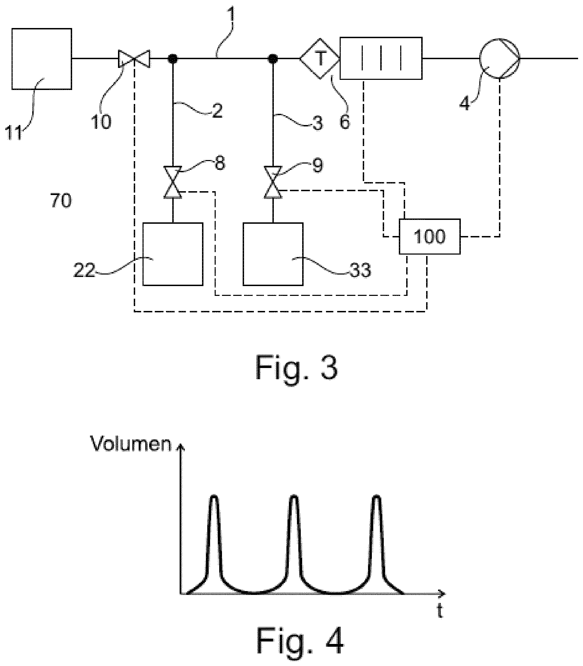

Fig. 3 zeigt eine dritte erfindungsgemäße Ausführungsform der Vorrichtung zur Herstellung von Dialysierflüssigkeit. Die Grundstruktur und die Referenzzeichen aus der ersten Ausführungsform bleiben unverändert, sodass im Folgenden nur auf Unterschiede der ersten und dritten Ausführungsform eingegangen wird. Die Förderungen des basischen Fluids und sauren Fluids erfolgt durch Induzieren eines Unterdrucks durch die Pumpe 4. Um einen Unterdruck zu erzeugen, wird das an der Hauptleitung 1 und vor der Mündungsstelle der Zuleitung 2 in die Hauptleitung 1 angeordnete steuerbare Ventil 10 geschlossen, oder zumindest der Durchflussquerschnitt der Hauptleitung 1 mittels eines geeigneten Ventils verringert. Beim Öffnen mindestens eines der Ventile 8, das an der Zuleitung 2 angeordnet ist, und 9, das an der Zuleitung 3 angeordnet ist, werden das basische Fluid und/oder saure Fluid durch den erzeugten Unterdruck zur Hauptleitung 1 hin gesaugt. Um das Wasser zu fördern wird das Ventil 10 geöffnet, sodass infolge des Unterdrucks das basische Fluid zur Pumpe 4 hin gesaugt wird. Durch eine Steuer- und Regeleinheit 100 werden die Signale der Messeinrichtung 6 verarbeitet und die Pumpe 4 sowie die Ventile 8, 9 und 10 gesteuert. Vorzugsweise wird der erzeugte Unterdruck auf -100 bis -150mmHg begrenzt, besonders bevorzugt ist die Unterdruckbegrenzung abhängig von der Höhendifferenz zwischen den Mündungsstellen der Zuleitung 2 und Zuleitung 3 in die Hauptleitung 1 und den Vorlagebehältern 22 und 33 des basischen Fluids und sauren Fluids.

Die Dosierung erfolgt bei der zweiten und dritten Ausführungsform dann im Zusammenspiel von (Dosier)pumpe und Ventilen so, dass wenn eines der Fluide gefördert und eingeleitet werden soll, die entsprechende (Dosier)pumpe betrieben wird und das Ventil an der Zuleitung des zu fördernden Fluids geöffnet ist. Das Ventil an der anderen Zuleitung bleibt währenddessen geschlossen. Sollen beide Fluide parallel zugegeben werden, kann dies ebenfalls durch die entsprechende Steuerung der Pumpe(n) und/oder Ventile erfolgen. Bei der Variante einer gemeinsamen (Dosier)pumpe für die Fluide in Kombination mit Ventilen an den beiden Zuleitung sind mehrere Betriebsarten möglich. In einer ersten Betriebsart kann die Förderrate der Pumpe für beide Fluidzugaben konstant gehalten werden, sodass die Ventile unterschiedliche Öffnungszeiten haben. In einer zweiten Betriebsart können die Öffnungszeiten der Ventile konstant gehalten werden und die Förderrate der Pumpe abhängig davon, welches Fluid gefördert werden soll, angepasst werden. In einer dritten Betriebsart sind die erste und zweite Betriebsart kombiniert, sodass sowohl die Pumpenförderrate, als auch die Ventilöffnungszeiten variiert werden können.

Fig. 4 zeigt ein Diagramm, das eine Zugabemenge (Volumen) in Abhängigkeit der Zeit (t) darstellt. Dabei handelt es sich um eine pulsierende bzw. stoßweise Förderung aus dem Stand der Technik, bei der die Zugabemengen von der basischen bzw. sauren Komponente nicht gleichmäßig, sondern gepulst eingespeist werden.

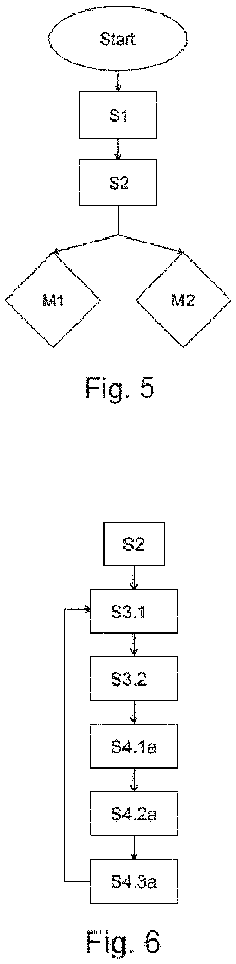

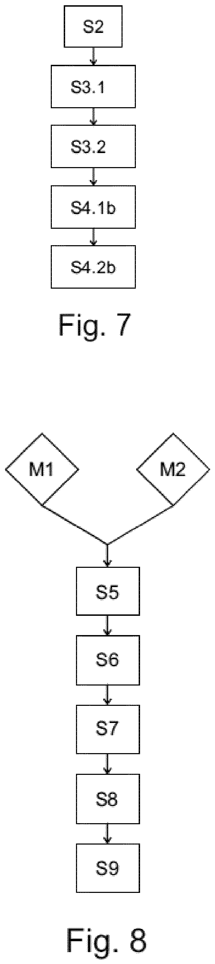

Fig. 5 zeigt eine schematische Darstellung des (Einmess-) Verfahrens gemäß einem Aspekt der Erfindung. Nachdem das Verfahren gestartet wurde, wird zunächst mindestens ein physikalischer und/oder chemischer Sollwert für ein Wasser-Fluid-Gemisch, das aus Wasser, dem basischen Fluid und/oder sauren Fluid besteht, definiert und die entsprechenden Förderraten des Wassers, des basischen Fluids und/oder sauren Fluids eingestellt. Die Einstellung kann durch Berechnung, analytische Ermittlung oder eine andere Definition erfolgen. Dies entspricht dem Verfahrensschritt S1. Anschließend wird Wasser mit der eingestellten Förderrate gefördert. Dies entspricht dem Verfahrensschritt S2. Daran anschließend werden entweder in einem Modus M1 oder in einem Modus M2 das basische Fluid und das saure Fluid gefördert und in das Wasser eingeleitet. Die Modi M1 und M2 sind nachstehend in den Beschreibungen der Fig. 6 und Fig. 7 näher erläutert.

Fig. 6 zeigt eine schematische Darstellung eines ersten erfindungsgemäßen Modus der Zudosierung M1, in dem das basische Fluid und das saure Fluid abwechselnd / seriell gefördert und zugegeben werden. Ausgehend vom Verfahrensschritt S2, der vorstehend in der Beschreibung der Fig. 5 näher erläutert wurde, wird nun die Förderung ausschließlich eines ersten aus den basischen und sauren Fluiden (nachfolgend ersten Fluid) begonnen und dieses in das geförderte Wasser eingeleitet. Dabei kann die Förderung mit einer eingestellten, insbesondere mit der in Schritt S1 eingestellten, Förderrate für das erste Fluid durchgeführt werden. Dies entspricht dem Verfahrensschritt S3.1. Anschließend wird mindestens ein physikalischer und/oder chemischer Parameter des Wasser-Fluid-Gemischs, das aus Wasser und dem ersten Fluid besteht oder diese enthält, gemessen und der Messwert mit mindestens einem definierten, insbesondere in Schritt S1 definierten, Sollwert für das Wasser-Fluid-Gemisch verglichen. Dies entspricht dem Verfahrensschritt S3.2. Im sich anschließenden Verfahrensschritt S4.1a wird nun bei Erreichen des mindestens einen Sollwerts das Fördern ausschließlich des ersten Fluids unterbrochen und das Fördern ausschließlich des zweiten aus den basischen und sauren Fluiden (nachfolgend zweites Fluid) begonnen und dieses in das geförderte Wasser eingeleitet. Dabei kann die Förderung mit einer eingestellten, insbesondere mit der in Schritt S1 eingestellten, Förderrate für das zweite Fluid durchgeführt werden. Anschließend wird mindestens ein physikalischer und/oder chemischer Parameter des Wasser-Fluid-Gemischs, das aus Wasser und dem sauren Fluid besteht oder diese enthält, gemessen und der Messwert mit dem mindestens einen definierten, insbesondere in Schritt S1 definierten, Sollwert für das Wasser-Fluid-Gemisch verglichen, was dem Verfahrensschritt S4.2a entspricht.

Im sich anschließenden Verfahrensschritt S4.3a wird nun bei Erreichen des mindestens einen Sollwerts das Fördern des zweiten Fluids unterbrochen und das Verfahren mit dem Schritt S3.1 fortgesetzt bzw. die Schritte ab Schritt S3.1 wiederholt. Soll die Förderrate für das Wasser, das erste Fluid und/oder das zweite Fluid geändert werden, kann dies durch Mess-Regelung zu jedem Verfahrenszeitpunkt erfolgen, in dem nur die Komponente, deren Förderrate geändert werden soll, gefördert wird.

Fig. 7 zeigt eine schematische Darstellung eines zweiten erfindungsgemäßen Modus der Zudosierung M2, in dem das basischen Fluid und das saure Fluid zeitversetzt parallel gefördert und zugeben werden. Der Modus M2 gleicht dem vorstehend beschriebenen Modus M1 bis einschließlich dem Schritt S3.2. Ausgehend vom Verfahrensschritt S3.2 wird bei dem sich anschließenden Verfahrensschritt S4.1b nun bei Erreichen des mindestens einen Sollwerts für das Wasser-Fluid-Gemisch, das aus Wasser und dem ersten Fluid besteht oder diese enthält, das Fördern des ersten Fluids mit der zuvor eingestellten Förderrate beibehalten und der zuletzt gemessene Parameter als Referenzwert behalten, worauf das Fördern des zweiten Fluids begonnen und dieses in das Wasser eingeleitet wird. Dabei kann die Förderung mit einer eingestellten, insbesondere mit der in Schritt S1 eingestellten, Förderrate für das zweite Fluid durchgeführt werden. Anschließend wird mindestens ein physikalischer und/oder chemischer Parameter des Wasser-Fluid-Gemischs, das aus Wasser und beiden besteht oder diese enthält, gemessen und der Messwert mit mindestens einem definierten, insbesondere in Schritt S1 definierten, Sollwert bzw. dem zuvor definierten Referenzwert verglichen, was dem Verfahrensschritt S4.2b entspricht.

Fig. 8 zeigt eine schematische Darstellung eines erfindungsgemäßen Modus M3 der Förderratenanpassung. Ausgehend von M2 soll die Förderrate eines ersten der basischen und sauren Fluide (nachfolgend erstes Fluid) geändert werden. Dazu wird im Anschluss an den Modus M2 zunächst das Fördern des zweiten der basischen und sauren Fluide (nachfolgend zweites Fluid) pausiert, was dem Schritt S5 entspricht. Anschließend wird das erste Fluid mit einer neu eingestellten Förderrate gefördert und in das Wasser-Fluid-Gemisch eingeleitet. Dies entspricht dem Schritt S6. Nachfolgend wird der Schritt S7 ausgeführt, in dem mindestens ein physikalischer und/oder chemischer Parameter der Wasser-Fluid-Mischung, die aus Wasser und dem ersten Fluid besteht oder diese enthält, gemessen und mit dem neuen, der geänderten Förderrate zugrunde liegenden, Sollwert verglichen. Anschließend wird bei Erreichen des neuen Sollwerts die neu eingestellte Förderung des ersten Fluids weiter betrieben und das Fördern des zweiten Fluids wieder aufgenommen und in das Wasser eingeleitet. Dies entspricht dem Schritt S8. Daran anschließend wird mindestens ein physikalischer und/oder chemischer Parameter des Fluid-Gemischs, das aus Wasser und den beiden Fluiden besteht oder diese enthält, gemessen und mit mindestens einem definierten Sollwert verglichen, was dem Schritt S9 entspricht. Anschließend kann das Verfahren im Modus M1, M2 oder M3 fortgesetzt werden. Alternativ kann im Schritt S5 das Fördern des zweiten Fluids unter Beibehaltung der eingestellten Förderrate weiter erfolgen. Die Schritte S7 und S8 entfallen in dieser Alternative.

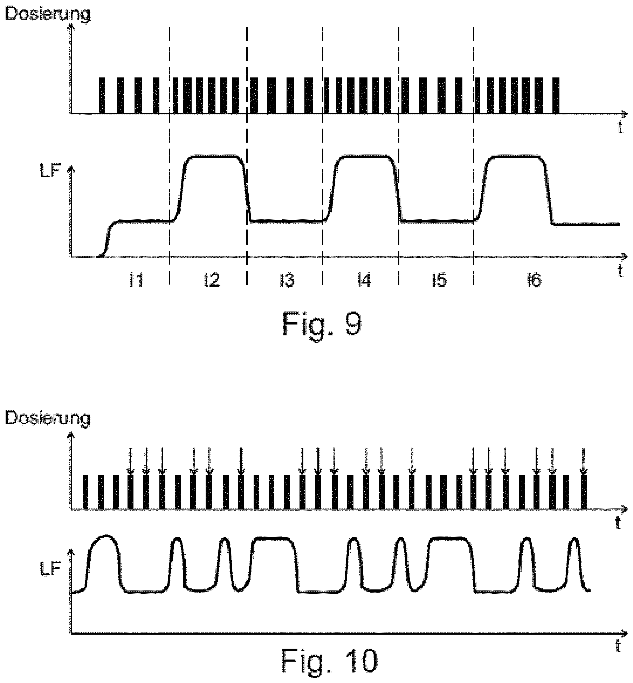

Fig. 9 zeigt ein Diagramm eines Dosierungs-/Zugabeverfahrens und ein Diagramm des aufgenommen Messsignals gemäß einem Aspekt der Erfindung. Dabei stellen im oberen Diagramm die Balken in den Intervallen I1, I3 und I5 die dosierten Volumina des basischen Fluids dar. Die Balken in den Intervallen I2, I4 und I6 stellen die dosierten Volumina des sauren Fluids dar. Anhand dieses Diagramms wird deutlich, dass die Dosierung der Fluide pulsierend erfolgt, die Fluidvolumina also stoßweise in das erste Fluid zugegeben werden.

Im unteren Diagramm ist die an einer Messeinrichtung gemessene Leitfähigkeit eines Wasser-Fluid-Gemischs, das aus Wasser, dem basischen und/oder sauren Fluid besteht, in Abhängigkeit der Zeit aufgetragen. Das basische Fluid weist eine geringe Leitfähigkeit auf als das saure Fluid. Wenn ein Volumen saures Fluid an der Messeinrichtung gemessen wird, fällt also das Leitfähigkeitssignal höher aus als wenn ein Volumen basisches Fluid an der Messeinrichtung gemessen wird. Deshalb ist in den Intervallen, in denen das saure Fluid pulsierend hinzugegeben wird, die gemessene Leitfähigkeit größer als in den Intervallen, in denen das basische Fluid hinzugegeben wird.

Fig. 10 zeigt ein Diagramm eines kodierten Dosierungs-/Zugabeverfahrens und ein Diagramm des aufgenommen Messsignals gemäß einem Aspekt der Erfindung. Dabei stellen die mit Pfeil markierten Balken im oberen Diagramm die dosierten Volumina des basischen Fluids und die Balken ohne Markierung die dosierten Volumina des sauren Fluids dar. Dabei ist die Kodierung, wie hier beschrieben, nicht auf das abwechselnde Fördern des basischen Fluids und sauren Fluids (z.B. im Modus M1) beschränkt, da die hohe Leitfähigkeit ("+1") auch erzielt werden kann, indem das Fördern des basischen Fluids beibehalten wird, wenn das saure Fluid hinzugegeben wird. Das Signal bzw. der Messwert des basischen Fluids kann entsprechend dem Barker-Code mit "-1" und das Signal bzw. der Messwert des sauren Fluids kann entsprechend dem Barker-Code mit "+1" kodiert werden. Das abgebildete Diagramm zeigt die Dosierung nach einem Barker-Code der Länge elf mit
"+1+1+1-1-1-1+1-1-1+1-1" bzw. "SK SK SK BK BK BK SK BK BK SK BK". Dabei kann "*SK*" auch "*SK* + *BK*" sein (z.B. im Modus M2).

Im unteren Diagramm ist die an einer Messeinrichtung gemessene Leitfähigkeit eines Fluid-Gemischs, das aus Wasser, dem basischen und/oder sauren Fluid besteht, in Abhängigkeit der Zeit aufgetragen. Das gemessene Signal entspricht dem Barker-Code der Länge elf mit

"+1+1+1-1-1-1+1-1-1+1-1" bzw. "SK SK SK BK BK BK SK BK BK SK BK", wobei "*SK*" auch "*SK* + *BK" sein* kann (z.B. im Modus M2).

Das an der Messeinrichtung resultierende Signal kann mit entsprechenden mathematischen Mitteln verarbeitet bzw. entfaltet werden, woraufhin Rückschlüsse auf die Zugabemengen der einzelnen Komponenten gezogen werden können. Das Gesamtsignal, beispielsweise die Gesamtleitfähigkeit, kann zusätzlich durch mathematische Mittelung ermittelt werden. Nach diesem Prinzip sind auch andere Kodierungen, wie sie z. B. in der Nachrichtentechnik angewandt werden, denkbar.

Aus der Signaldynamik kann auch geschlossen werden, ob die Funktion der Messeinrichtung noch gegeben ist. Dadurch, dass zu Beginn einer Aufbereitungsphase eine Kalibrierung auf die einzelnen Fluide durchgeführt wird, ist ein Erwartungswert für einen Unterschied und ein Verhältnis der beiden Parameter der Fluide, beispielsweise Leitfähigkeit, bekannt. Durch die mathematische Entfaltung können ständig beide Messwerte, also für ein Gemisch aus dem basischen Fluid und dem sauren Fluid, berechnet werden und zueinander ins Verhältnis gesetzt werden. Aus dem Abgleich mit dem ursprünglichen Verhältnis kann geschlossen werden, dass die Messeinrichtung noch funktioniert. Mit diesem Verfahren lässt sich auch in Vorrichtungen aus dem Stand der Technik eine vorhandene zusätzliche Messeinrichtung einsparen.

## Patentansprüche

1. Verfahren zur Herstellung von Dialysierflüssigkeit für den Einsatz bei einer extrakorporalen Blutbehandlung an einer Vorrichtung zur extrakorporalen Blutbehandlung mit den Schritten
Fördern von Wasser, vorzugsweise osmotisches oder hochreines Wasser, über eine Hauptleitung (1) (S2);
Fördern eines basischen Fluids und Einleiten an einer Einleitstelle in die Hauptleitung (1);
Fördern eines sauren Fluids und Einleiten an einer Einleitstelle in die Hauptleitung (1);
Messen mindestens eines physikalischen und/oder chemischen Parameters des Wasser-Fluid-Gemischs, das aus Wasser, dem sauren Fluid und/oder dem basischen Fluid besteht, mittels einer ersten Messeinrichtung (6) (S.3.2); und
Einstellen der Förderraten für das Wasser, das saure Fluid und/oder das basische Fluid durch die Steuer- und Regeleinheit (100) in Abhängigkeit vom erfassten Messwert,
wobei
das Messen des mindestens einen physikalischen und/oder chemischen Parameters mittels einer Messeinrichtung (6) erfolgt, die an einem Abschnitt der Hauptleitung (1), der jeweils stromab zu der Einleitstelle für das saure Fluid und zu der Einleitstelle für das basische Fluid liegt, angeordnet ist, **dadurch gekennzeichnet, dass**
die Steuer- und Regeleinheit (100) das Einleiten des sauren Fluids und des basischen Fluids zumindest temporär so steuert, dass über einen vorbestimmten Zeitraum oder ein vorbestimmtes Intervall nur ein erstes Fluid aus den sauren und basischen Fluiden in die Hauptleitung (1) eingeleitet wird und zunächst der Parameter des Wasser-Fluid-Gemischs mit nur dem ersten Fluid gemessen und basierend darauf die Dosierung oder Förderrate des ersten Fluids bestimmt wird; daraufhin die Einleitung des ersten Fluids unterbrochen und stattdessen ein zweites Fluid aus den sauren und basischen Fluiden eingeleitet wird, wobei der Parameter des Wasser-Fluid-Gemischs mit nur dem zweiten Fluid gemessen und basierend darauf die Dosierung oder Förderrate des zweiten Fluids bestimmt wird und schließlich beide Fluide mit den zuvor bestimmten Dosierungen oder Förderraten eingeleitet werden.

2. Verfahren nach Anspruch 1, **dadurch gekennzeichnet, dass** das Fördern und Einleiten des basischen Fluids mit der zuvor bestimmten Dosierung oder Förderrate und das Fördern und Einleiten des sauren Fluids mit der zuvor bestimmten Dosierung oder Förderrate abwechselnd, insbesondere kontinuierlich abwechselnd, erfolgt.

3. Verfahren nach Anspruch 1, **dadurch gekennzeichnet, dass** das Fördern und Einleiten des basischen Fluids mit der zuvor bestimmten Dosierung oder Förderrate und das Fördern und Einleiten des sauren Fluids mit der zuvor bestimmten Dosierung oder Förderrate zeitversetzt parallel erfolgt.

4. Verfahren nach einem der Ansprüche 1 bis 3, **dadurch gekennzeichnet, dass** bei einer Anpassung einer Förderrate eines Fluids aus den basischen und sauren Fluiden für den Zeitraum des Anpassens, insbesondere von der Änderung der Förderrate bis zum Erreichen des neuen Sollwerts des Wasser-Fluid-Gemischs, die vorder Anpassung eingestellte Förderrate des anderen Fluids aus den basischen und sauren Fluiden beibehalten oder dessen Förderung pausiert wird.

5. Verfahren nach einem der Ansprüche 1 bis 4, **dadurch gekennzeichnet, dass** das Wasser-Fluid-Gemisch bei einer Änderungen oder neuen Einstellung mindestens eines Sollwerts bis zum wenigstens erstmaligen Erreichen des Sollwerts an der Vorrichtung zur extrakorporalen Blutbehandlung vorbeigeführt wird.

6. Verfahren nach einem der Ansprüche 1 bis 5, **dadurch gekennzeichnet, dass** in einem Fall, in dem zum Erreichen mindestens eines Sollwerts des Wasser-Fluid-Gemischs eine definierte Zeitspanne überschritten wird und/oder eine Förderrate eingestellt wird, die einen definierten Grenzwert überschreitet, ein Warnhinweis an der Vorrichtung zur extrakorporalen Blutbehandlung ausgegeben wird.

7. Verfahren nach einem der Ansprüche 1 bis 6, **dadurch gekennzeichnet, dass** das Verfahren weiter einen Schritt
Messen mindestens eines physikalischen und/oder chemischen Parameters des Wasser-Fluid-Gemischs, das aus Wasser, dem basischen Fluid und/oder dem sauren Fluid besteht, mittels einer weiteren Messeinrichtung, vorzugsweise eine Leitfähigkeitssonde, die an der Hauptleitung (1) unmittelbar stromab zu der ersten Messeinrichtung (6) angeordnet ist,
aufweist; und
dass in einem Fall, in dem eine Abweichung zwischen dem an der ersten Messeinrichtung (6) und dem an der weiteren Messeinrichtung gemessenen Parameter einen definierten Grenzwert überschreitet, ein Warnhinweis an der Vorrichtung zur extrakorporalen Blutbehandlung ausgegeben wird.

8. Verfahren nach einem der Ansprüche 1 bis 7, **dadurch gekennzeichnet, dass** das Wasser-Fluid-Gemisch, das aus Wasser, dem basischen Fluid und/oder dem sauren Fluid besteht, mittels mindestens einer Mischeinrichtung, vorzugsweise einem statischer Mischer, durchmischt wird.

9. Verfahren nach einem der Ansprüche 1 bis 8, **dadurch gekennzeichnet, dass** die Mischeinrichtung eine Kammer eines kammerbasierten Bilanzierungssystems ist und der physikalische und/oder chemische Sollwert eines Wasser-Fluid-Gemischs so definiert ist, dass er rechnerisch innerhalb einer Zeiteinheit, die höchstens einer Bilanzkammerumschaltung entspricht, realisiert werden kann.

10. Verfahren nach einem der Ansprüche 1 bis 9, **dadurch gekennzeichnet, dass** die Zugabe des basischen Fluids und des sauren Fluids kodiert wird, vorzugsweise durch Verwendung eines Barker-Codes.

11. Vorrichtung (50; 60; 70) zur Herstellung von Dialysierflüssigkeit, die die drei Komponenten Wasser und basisches Fluid und saures Fluid enthält, für den Einsatz bei einer extrakorporalen Blutbehandlung mit:
einer Hauptleitung (1) zur Zufuhr von Wasser, vorzugweise osmotisches oder hochreines Wasser, in deren Verlauf ein saures und ein basisches Fluid in jeweils einer bestimmten Dosierung eingeleitet wird, wobei die Dosierung von einer Steuer- und Regeleinheit (100) in Abhängigkeit von mindestens einem chemischen und/oder physikalischen Parameter, vorzugsweise Leitfähigkeit, des Wasser-Fluid-Gemischs eingestellt wird und der mindestens eine chemische und/oder physikalische Parameter von einer Messeinrichtung (6), insbesondere einer Leitfähigkeitsmesssonde, erfasst wird, wobei
eine erste, vorzugweise einzige, Messeinrichtung (6) an einem Abschnitt der Hauptleitung (1), der jeweils stromab zu der Einleitstelle für das saure Fluid und zu der Einleitstelle für das basische Fluid liegt, angeordnet ist und die Steuer- und Regeleinheit (100) die Einleitung des sauren und basischen Fluids zumindest temporär so steuert, dass über einen vorbestimmten Zeitraum oder ein vorbestimmtes Intervall nur eines aus den sauren und basischen Fluiden in die Hauptleitung (1) eingeleitet wird, **dadurch gekennzeichnet, dass** die Vorrichtung (50; 60; 70) dazu vorgesehen und angepasst ist, das Verfahren nach einem der Ansprüche 1 bis 10 durchzuführen.

12. Vorrichtung (50; 60) nach Anspruch 11, **dadurch gekennzeichnet, dass** die Vorrichtung (50; 60) wenigstens eine, vorzugsweise einzige, Pumpe (5a; 5b; 5c) aufweist, die dazu vorgesehen und angepasst ist, das saure Fluid und/oder das basische Fluid zur Hauptleitung (1) hin zu fördern.

13. Vorrichtung (60; 70) nach einem der vorangehenden Ansprüche 11 und 12, **dadurch gekennzeichnet, dass** das Einleiten des sauren Fluids und/oder das Einleiten des basischen Fluids über mindestens ein Ventil (8, 9), vorzugsweise ein Mehrwegeventil, erfolgt und insbesondere an der Hauptleitung (1) ein Ventil (10), vorzugsweise ein Druckregelventil, vorgesehen ist, das insbesondere jeweils stromauf von der Einleitstelle für das saure Fluid und von der Einleitstelle für das basische Fluid angeordnet ist.

14. Vorrichtung (50; 60; 70) nach einem der vorangehenden Ansprüche 11 bis 13, **dadurch gekennzeichnet, dass** eine weitere Messeinrichtung unmittelbar stromab zu der ersten Messeinrichtung (6) vorgesehen und dazu angepasst ist, mindestens einen physikalischen und/oder chemischen Parameter des die Hauptleitung (1) durchströmenden Wasser-Fluid-Gemischs zu erfassen.

15. Vorrichtung (50; 60; 70) nach einem der vorangehenden Ansprüche 11 bis 14, **dadurch gekennzeichnet, dass** die Vorrichtung mindestens eine Mischeinrichtung, vorzugsweise einen statischer Mischer oder eine Kammer eines kammerbasierten Bilanzierungssystems, aufweist und die Mischeinrichtung vorzugsweise stromab zu der ersten und/oder weiteren Messeinrichtung und weiter vorzugsweise jeweils stromab zu der Einleitstelle für das saure Fluid und zu der Einleitstelle für das basische Fluid angeordnet ist.

## Claims

1. Method for producing dialysis fluid for use in extracorporeal blood treatment on an apparatus for extracorporeal blood treatment, comprising the steps of
delivering water, preferably osmotic or high-purity water, via a main line (1) (S2);
delivering a basic fluid and introducing the basic fluid at a feeding point into the main line (1);
delivering an acidic fluid and introducing the acidic fluid at a feeding point into the main line (1);
measuring at least one physical and/or chemical parameter of the water-fluid mixture consisting of water, the acidic fluid and/or the basic fluid by means of a first measuring device (6) (S.3.2); and
adjusting the delivery rates for the water, the acid fluid and/or the basic fluid by a control and regulating unit (100) as a function of the measured value detected,
wherein
the measuring of the at least one physical and/or chemical parameter is carried out by means of a measuring device (6) arranged at a section of the main line (1) located downstream of the feeding point for the acidic fluid and the feeding point for the basic fluid, respectively, **characterized in that**
the control and regulating unit (100) controls at least temporarily the feeding of the acid fluid and the basic fluid in such a way that, over a predetermined period of time or a predetermined interval, only a first fluid from among the acid and basic fluids is fed into the main line (1), and at first the parameter of the water-fluid mixture containing only the first fluid is measured and, based thereon, the dosage or delivery rate of the first fluid is determined;
thereupon the feeding of the first fluid is interrupted and instead a second fluid from among the acidic and basic fluids is fed, wherein the parameter of the water-fluid mixture with only the second fluid is measured and based thereon the dosage or delivery rate of the second fluid is determined and finally both fluids are fed with the afore-determined dosages or delivery rates.

2. Method according to claim 1, **characterized in that** the delivering and feeding of the basic fluid at the afore-determined dosage or delivering rate and the delivering and feeding of the acidic fluid at the afore-determined dosage or delivering rate are carried out alternately, in particular continuously alternated.

3. Method according to claim 1, **characterized in that** the delivery and feeding of the basic fluid at the afore -determined dosage or delivery rate and the delivery and feeding of the acidic fluid at the afore-determined dosage or delivery rate are carried out time-shifted in parallel.

4. Method according to any one of claims 1 to 3, **characterized in that**, while adjusting a delivery rate of a fluid extracorporeal the basic and acidic fluids, for the period of the adjustment, in particular of the change in the delivery rate until the new target value of the water-fluid mixture is reached, the delivery rate of the other fluid from among the basic and acidic fluids set prior to the adjustment is maintained or delivery thereof is suspended.

5. Method according to one of claims 1 to 4, **characterized in that** the water-fluid mixture is past by the apparatus for extracorporeal blood treatment while a change or new adjustment of at least one target value until the target value is reached at least for the first time.

6. Method according to any one of claims 1 to 5, **characterized in that** in a case in which, for reaching at least one target value of the water-fluid mixture, a defined time span is exceeded and/or a delivery rate is set that exceeds a defined limit value is set, a warning is output at the apparatus for extracorporeal blood treatment.

7. Method according to any one of claims 1 to 6, **characterized in that** the method further comprises a step of
measuring at least one physical and/or chemical parameter of the water-fluid mixture consisting of water, the basic fluid and/or the acidic fluid by means of a further measuring device, preferably a conductivity probe, arranged on the main line (1) immediately downstream of the first measuring device (6),
and
**in that** in a case in which a deviation between the parameter measured at the first measuring device (6) and the parameter measured at the further measuring device exceeds a defined limit value, a warning is output at the apparatus for extracorporeal blood treatment.

8. Method according to one of claims 1 to 7, **characterized in that** the water-fluid mixture consisting of water, the basic fluid and/or the acidic fluid is mixed by means of at least one mixing device, preferably a static mixer.

9. Method according to one of claims 1 to 8, **characterized in that** the mixing device is a chamber of a chamber-based balancing system and the physical and/or chemical target value of a water-fluid mixture is defined in such a way that it can be realized by calculation within a time unit corresponding at most to a balancing chamber switching.

10. Method according to any one of claims 1 to 9, **characterized in that** the addition of the basic fluid and the acidic fluid is encoded, preferably by using a Barker code.

11. Apparatus (50; 60; 70) for preparing dialysis fluid containing the three components of water and basic fluid and acidic fluid, for use in extracorporeal blood treatment, comprising:
a main line (1) for the supply of water, preferably osmotic or high-purity water, in the course of which each of an acidic fluid and a basic fluid are fed at a specific dosage, wherein the dosage is set by a control and regulating unit (100) as a function of at least one chemical and/or physical parameter, preferably conductivity, of the water-fluid mixture, and the at least one chemical and/or physical parameter being detected by a measuring device (6), in particular a conductivity measuring probe, wherein
a first, preferably single, measuring device (6) is arranged at a section of the main line (1) located downstream of the feeding point for the acidic fluid and of the feeding point for the basic fluid, respectively, and the control and regulating unit (100) controls the feeding of the acidic and basic fluids at least temporarily in such a way that only one of the acidic and basic fluids is fed into the main line (1) over a predetermined period of time or a predetermined interval, **characterized in that** the apparatus (50; 60; 70) is provided and adapted to perform the method according to any one of claims 1 to 10.

12. Apparatus (50; 60) according to claim 11, **characterized in that** the apparatus (50; 60) comprises at least one, preferably single, pump (5a; 5b; 5c) which is provided and adapted to deliver the acidic fluid and/or the basic fluid towards the main line (1).

13. Apparatus (60; 70) according to one of the preceding claims 11 and 12, **characterized in that** the feeding of the acid fluid and/or the introduction of the basic fluid takes place via at least one valve (8, 9), preferably a multi-way valve, and in particular a valve (10), preferably a pressure regulating valve, is provided on the main line (1), which valve is arranged in particular in each case upstream of the feeding point for the acid fluid and of the feeding point for the basic fluid.

14. Apparatus (50; 60; 70) according to one of the preceding claims 11 to 13, **characterized in that** a further measuring device is provided immediately downstream of the first measuring device (6) and is adapted to detect at least one physical and/or chemical parameter of the water-fluid mixture flowing through the main line (1).

15. Apparatus (50; 60; 70) according to one of the preceding claims 11 to 14, **characterized in that** the device comprises at least one mixing device, preferably a static mixer or a chamber of a chamber-based balancing system, and the mixing device is preferably arranged downstream of the first and/or further measuring device and further preferably in each case downstream of the feeding point for the acidic fluid and of the feeding point for the basic fluid.

## Revendications

1. Procédé de fabrication d'un liquide de dialyse pour un emploi lors d'un traitement extracorporel de sang au niveau d'un dispositif pour le traitement extracorporel de sang avec les étapes de
acheminement d'une eau, de préférence une eau osmotique ou ultra-pure, via une conduite principale (1) (S2) ;
acheminement d'un fluide basique et introduction, au niveau d'un emplacement d'introduction, dans la conduite principale (1) ;
acheminement d'un fluide acide et introduction, au niveau d'un emplacement d'introduction, dans la conduite principale (1) ;
mesure d'au moins un paramètre physique et/ou chimique du mélange eau/fluide, qui se compose d'eau, du fluide acide et/ou du fluide basique, au moyen d'un premier équipement de mesure (6) (S.3.2) ; et
réglage des débits d'acheminement pour l'eau, le fluide acide et/ou le fluide basique par l'unité de commande et de réglage (100) en fonction de la valeur mesurée saisie,
dans lequel
la mesure du au moins un paramètre physique et/ou chimique est réalisé au moyen d'un équipement de mesure (6) qui est agencé au niveau d'une section de la conduite principale (1), laquelle section se trouve respectivement en aval de l'emplacement d'introduction pour le fluide acide et de l'emplacement d'introduction pour le fluide basique, **caractérisé en ce que**
l'unité de commande et de réglage (100) commande au moins temporairement l'introduction du fluide acide et du fluide basique de sorte que, via un espace de temps prédéterminé ou un intervalle prédéterminé, seul un premier fluide parmi les fluides acide et basique soit introduit dans la conduite principale (1) et le paramètre du mélange eau/fluide soit d'abord mesuré uniquement avec le premier fluide et sur cette base le dosage ou le débit d'acheminement du premier fluide soit déterminé ;
l'introduction du premier fluide soit ensuite interrompue et à la place soit introduit un second fluide parmi les fluides acide et basique, dans lequel le paramètre du mélange eau/fluide soit mesuré uniquement avec le second fluide et sur cette base le dosage ou le débit d'acheminement du second fluide soit déterminé et finalement les deux fluides soient introduits avec les dosages ou les débits d'acheminement déterminés auparavant.

2. Procédé selon la revendication 1, **caractérisé en ce que** l'acheminement et l'introduction du fluide basique avec le dosage ou le débit d'acheminement déterminé auparavant et l'acheminement et l'introduction du fluide acide avec le dosage ou le débit d'acheminement déterminé auparavant se produit en alternance, en particulier continuellement en alternance.

3. Procédé selon la revendication 1, **caractérisé en ce que** l'acheminement et l'introduction du fluide basique avec le dosage ou le débit d'acheminement déterminé auparavant et l'acheminement et l'introduction du fluide acide avec le dosage ou le débit d'acheminement déterminé auparavant se produit parallèlement avec un décalage temporel.

4. Procédé selon l'une des revendications 1 à 3, **caractérisé en ce que** lors d'un ajustement d'un débit d'acheminement d'un fluide parmi les fluides basique et acide pour l'espace de temps de l'ajustement, en particulier depuis la modification du débit d'acheminement jusqu'à l'obtention de la nouvelle valeur de consigne du mélange eau/fluide, le débit d'acheminement, réglé avant l'ajustement, de l'autre fluide parmi les fluides basique et acide est maintenu ou l'acheminement de celui-ci est mis en pause.

5. Procédé selon l'une des revendications 1 à 4, **caractérisé en ce que** le mélange eau/fluide, lors d'une modification ou d'un nouveau réglage d'au moins une valeur de consigne, est passé devant le dispositif pour le traitement extracorporel de sang jusqu'à obtenir au moins pour la première fois la valeur de consigne.

6. Procédé selon l'une des revendications 1 à 5, **caractérisé en ce que** dans le cas où, pour obtenir au moins une valeur de consigne du mélange eau/fluide, un laps de temps défini est dépassé et/ou un débit d'acheminement qui dépasse une valeur seuil définie est réglé, un avertissement est transmis au dispositif pour le traitement extracorporel de sang.

7. Procédé selon l'une des revendications 1 à 6, **caractérisé en ce que** le procédé présente en outre une étape de
mesure d'au moins un paramètre physique et/ou chimique du mélange eau/fluide, qui se compose d'eau, du fluide basique et/ou du fluide acide, au moyen d'un autre équipement de mesure, de préférence une sonde de conductivité, qui est agencé au niveau de la conduite principale (1), directement en aval du premier équipement de mesure (6) ;
et
**en ce que** dans le cas où un écart entre le paramètre mesuré au niveau du premier équipement de mesure (6) et celui mesuré au niveau de l'autre équipement de mesure dépasse une valeur seuil définie, un avertissement est transmis au dispositif pour le traitement extracorporel de sang.

8. Procédé selon l'une des revendications 1 à 7, **caractérisé en ce que** le mélange eau/fluide, qui se compose d'eau, du fluide basique et/ou du fluide acide, est mélangé au moyen d'au moins un équipement de mélange, de préférence un mélangeur statique.

9. Procédé selon l'une des revendications 1 à 8, **caractérisé en ce que** l'équipement de mélange est une chambre d'un système d'équilibrage fondé sur une chambre et la valeur de consigne physique et/ou chimique d'un mélange eau/fluide est définie de sorte qu'elle puisse être réalisée par voie de calcul à l'intérieur d'une unité de temps qui correspond au plus à un basculement de chambre d'équilibrage.

10. Procédé selon l'une des revendications 1 à 9, **caractérisé en ce que** l'ajout du fluide basique et du fluide acide est codé, de préférence en utilisant un code Barker.

11. Dispositif (50 ; 60 ; 70) de fabrication d'un liquide de dialyse qui contient les trois composantes eau et fluide basique et fluide acide, pour un emploi lors d'un traitement extracorporel de sang avec :
une conduite principale (1) pour l'amenée d'eau, de préférence une eau osmotique ou ultra-pure, dans le parcours de laquelle est introduit un fluide acide et un fluide basique dans un dosage respectif déterminé, dans lequel le dosage du mélange eau/fluide est réglé par une unité de commande et de réglage (100) en fonction d'au moins un paramètre chimique et/ou physique, de préférence une conductivité, et le au moins un paramètre chimique et/ou physique est saisi par un équipement de mesure (6), en particulier une sonde de conductivité, dans lequel
un premier, de préférence unique, équipement de mesure (6) est agencé au niveau d'une section de la conduite principale (1) qui se trouve respectivement en aval de l'emplacement d'introduction pour le fluide acide et de l'emplacement d'introduction pour le fluide basique, et l'unité de commande et de réglage (100) commande au moins temporairement l'introduction du fluide acide et basique de sorte que, via un espace de temps prédéterminé ou un intervalle prédéterminé, seul l'un parmi les fluides acide et basique soit introduit dans la conduite principale (1), **caractérisé en ce que** le dispositif (50 ; 60 ; 70) est prévu et ajusté pour exécuter le procédé selon l'une des revendications 1 à 10.

12. Dispositif (50 ; 60) selon la revendication 11, **caractérisé en ce que** le dispositif (50 ; 60) présente au moins une, de préférence unique, pompe (5a ; 5b ; 5c) qui est prévue et ajustée pour acheminer le fluide acide et/ou le fluide basique en allant vers la conduite principale (1).

13. Dispositif (60 ; 70) selon l'une des revendications précédentes 11 et 12, **caractérisé en ce que** l'introduction du fluide acide et/ou l'introduction du fluide basique se produit via au moins une vanne (8,9), de préférence une vanne à plusieurs voies, et une vanne (10), de préférence une vanne de régulation de pression, est en particulier prévue au niveau de la conduite principale (1), laquelle est agencée en particulier respectivement en amont de l'emplacement d'introduction pour le fluide acide et de l'emplacement d'introduction pour le fluide basique.

14. Dispositif (50 ; 60 ; 70) selon l'une des revendications précédentes 11 à 13, **caractérisé en ce qu'**un autre équipement de mesure est prévu directement en aval du premier équipement de mesure (6) et ajusté pour saisir au moins un paramètre physique et/ou chimique du mélange eau/fluide qui s'écoule à travers la conduite principale (1).

15. Dispositif (50 ; 60 ; 70) selon l'une des revendications précédentes 11 à 14, **caractérisé en ce que** le dispositif présente au moins un équipement de mélange, de préférence un mélangeur statique ou une chambre d'un système d'équilibrage fondé sur une chambre, et l'équipement de mélange est agencé de préférence en aval du premier et/ou de l'autre équipement de mesure et davantage de préférence respectivement en aval de l'emplacement d'introduction pour le fluide acide et de l'emplacement d'introduction pour le fluide basique.
